# EUROPEAN PATENT SPECIFICATION

(11) **EP 1 301 790 B1**
(45) Date of publication and mention of the grant of the patent: **09.04.2008**
(21) Application number: 01974080.2
(22) Date of filing: 11.07.2001
(51) Int. Cl.: G01N 33/543, G01N 33/68, G01N 33/558, G01N 33/74, G01N 33/70, G01N 33/573

(54) **DIRECT ASSESSMENT OF ANALYTE TO REFERENCE MOLECULE RATIOS**
DIREKTE BESTIMMUNG DES QUOTIENTS VON ANALYT- ZU REFERENZMOLEKÜLEN
EVALUATION DIRECTE DE RAPPORTS ANALYTE/MOLECULE DE REFERENCE

(30) Priority: 12.07.2000 EP 00115162
(43) Date of publication of application: 16.04.2003
(73) Proprietor: Naser, Werner, Dr., 82377 Penzberg (DE)
(72) Inventor: NASER, Werner, 82377 Penzberg (DE); GOERLACH-GRAW, Ada, 67229 Grosskarlbach (DE)
(86) International application number: PCT/EP2001/007986
(87) International publication number: WO 2002/004950

(56) References cited:
- WO-A-95/13543
- WO-A-95/20764
- WO-A-97/08551
- WO-A-98/52041
- J. M. BLATT ET AL.: "A miniaturized, self-contained, single-use, disposable assay device for the quantitative determination of the bone resorption marker NTx, in urine" CLINICAL CHEMISTRY, vol. 44, no. 9, September 1998 (1998-09), pages 2051-2052, XP002157132 WINSTON US cited in the application

### SEQUENCE LISTING

<110> Naser Dr., Werner
<120> Direct Assessment of Analyte to Reference Molecule Ratios
<130> 1008/00/EP
<140>
   <141>
<160> 3
<170> PatentIn Ver. 2.1
<210> 1
   <211> 7
   <212> PRT
   <213> Homo sapiens
<400> 1
   Asp Glu Lys Ser Thr Gly Gly
     1               5
<210> 2
   <211> 8
   <212> PRT
   <213> Homo sapiens
<400> 2
   Gln Tyr Asp Gly Lys Gly Val Gly
     1               5
<210> 3
   <211> 8
   <212> PRT
   <213> Homo sapiens
<400> 3
   Glu Lys Ala His Asp Gly Gly Arg
     1               5

## Description

### Field of the invention:

The invention relates to reagents, methods and devices for the direct assessment of analytes of interest in relation to a reference molecule, wherein the concentrations of both the analyte as well as the reference molecule are not determined individually. The result of measurements according to the present invention is directly or indirectly correlated to the analyte to reference molecule ratio as present in the sample investigated.

### Background of the invention:

Existing methods for determining the ratios of biological molecules involve multiple analysis steps and frequently require a large amount of time to carry them out. Most frequently the concentrations of two or more molecules are determined by independent procedures and analyte to analyte, or analyte to reference molecule ratios are calculated based on the individual measurements. This means that two or more different and independent assays have to be performed in order to determine the ratio(s) of (an) analyte(s) to one or more reference molecules.

There are in-numerous ways of determining analyte concentrations in biological and medical samples. Frequently analytes are detected by means of specific binding reactions. Amongst the specific binding reactions the binding of antibodies to their antigens is the best known example.

Radioimmunoassays (RIAs), enzyme-linked immunosorbent assays (ELISAs), enzyme immunoassays based on fluorescence, turbidimetric assays, as well as assays being performed on test strips have become widely known and applied in routine analysis and diagnostics. Such assay systems are described in numerous scientific papers, patents and text books. All basic procedures pertinent to that field are well-known to the expert in the field.

Ratios of analytes are determined wherever the absolute values of single analytes are of less diagnostic or prognostic significance as compared to relative concentrations or ratios of at least two different types of molecules. Amongst the well known examples of clinically relevant ratios of analytes measured from blood, serum or plasma two shall be mentioned: The %-HbA1c (this is the ratio of glycated haemoglobin to the non-glycated molecule - it gives important information for improved management of diabetics); the free to total PSA (free prostate specific antigen (PSA) to total PSA- very important to differentiate benign hyperplasia from prostate tumours).

The above ratios are determined, from serum, plasma or blood. All of which are considered to be rather a constant biological sample, especially, as compared to samples like urine, saliva, exudate fluid, synovia, cerebrospinal fluid and tissue extracts. Such samples have proven to be highly variable no matter whether the analyte measured is a salt, glucose, a small molecule like a steroid hormone, a protein, a peptide, an antibody, RNA or DNA.

Values for (an) analyte(s) measured in these highly variable samples can only be compared to one another and relevant information thus be only obtained if the values measured in such samples are normalised to (a) reference molecule(s). Such a reference molecule, e.g. creatinine as an indicator for the relative concentration of urine samples, total protein for cerebrospinal fluid or the (RNA-) DNA-content, e.g. of a (house-keeping) gene as an indicator for efficiency of extraction, has to be measured in addition to the analyte under investigation. Comparability of analyte values derived from such samples is only given after they have been normalised and expressed in amounts or concentrations relative to one and the same reference molecule, e.g. per mg or mmol creatinine.

In recent years several approaches have been described and disclosed, enabling the direct detection of analyte-derivative to analyte ratios or vice versa. In this context the term analyte-derivative shall indicate that it is a biologically modified form of the analyte (a derivative), which is investigated in relation to total analyte (derivative and unmodified) or unmodified analyte alone. All these methods require binding of both (or if more of all) variants of this analyte to a single binding structure, capable of binding both (or all) variants of the analyte under investigation.

One recent example of such improvements is given in WO 98/52041, which is entitled "Rapid evaluation of the ratio of biological molecules". In essence WO 98/52041 describes that it is possible to coat a solid support with a first binding partner to an analyte and its derivative(s) and to specifically detect only the derivative(s) of interest. When, e. g., an antibody to haemoglobin is fixed to a solid support and analyte plus analyte derivative are present in rather a high concentration, all anti-haemoglobin antibodies bind to either haemoglobin or its derivative. Detection can then be performed for either haemoglobin or the derivative, and the signal generated then corresponds e.g. to the ratio of this analyte-derivative to the total analyte in the sample. Specific examples are given for % HbA1c or for oxidised or reduced troponin I.

To summarise all methodologies for determination of analyte ratios known in the art require, either the independent determination of at least two different molecules (e.g. the analyte and another analyte and/or a reference molecule) or the most advanced ones, as described above, that analyte and its derivatives or variants are related to each other and exhibit similar binding properties to one of the specific binding partners used and that analyte and analyte derivative are present in very high concentrations in the sample.

The situation is completely different in case the analyte under investigation and the reference molecule are not related to each other, as e.g. for the normalisation of urinary analytes to creatinine. Nothing has been found in the state of the art, disclosing the direct measurement of an analyte to reference molecule ratio. All presently known methods require the independent determination of both the analyte(s) and of at least one reference molecule.

Samples like urine are quite easily obtained and thus would be ideal wherever a large number of individuals is to be tested. One special example for such a potential application is in the screening for osteoporotic conditions or osteoporotic risk.

To date several approaches to diagnose osteoporosis by aid of urinary samples are under investigation. Most advanced markers are the so-called collagen degradation products (CDPs), which all have to be normalised for volume effects (concentration/dilution) with urine as sample, e.g., by using creatinine as a reference molecule or by use of 24h-collections.

It has now surprisingly been found that analyte to reference molecule ratios can be directly measured, obviating the need for several independent measurements. This greatly enhances use and applicability of sample sources, which otherwise are difficult to handle due to their inherent high variability.

In addition the methods and devices of the present invention have the striking advantage that the sample volume applied may be varied as required. It is not necessary to apply exactly known amounts (volumes) of sample or to use devices which are designed to accept a predetermined volume of sample.

The methods and devices of the invention developed to detect analyte to reference molecule ratios can be used with great advantage wherever the analysis will be performed by non-skilled personal or by the customer himself. This especially true for applications in urinalysis, and therein especially in areas like pregnancy testing, testing for albumin in urine, assessment of ovulation cycle or screening for osteoporosis.

As indicated, the diagnostic use of easily obtainable samples, like sputum, tear liquid, exudate, or urine is severely hampered by the fact that correction measures are a must for any (semi-) quantitative analysis using such samples. Only where qualitative analysis, a simple yes or no result, is sufficient, these sample sources are widely used, e.g. to detect the abuse of drugs.

When analytes are quantified from urine, e.g. by dry chemistry or test strip devices. The volume applied to the strip has to be controlled, e.g., by applying only the required volume or amount of sample to the device, or by using devices which are designed to only take a defined volume of sample, e.g. by providing for application zones, which take a defined volume of sample, and/or by manufacturing the whole device to only analyse such a defined volume. It is only this way possible to determine the concentration of an analyte by a dry chemistry device, e. g. by aid of a test strip.

In order to compensate for concentration/dilution effects of the sample investigated, however, correction measures still have to be used to correct for sample variability. This problem is also obviated by the present invention. Using the procedures reagents and devices disclosed, it is neither necessary to apply exactly controlled volumes of a sample to the device, nor is it required to measure the concentrations of both reference molecule and analyte.

The procedures and devices, as described in the present invention largely improve on measurement of analytes to reference molecule ratios from highly variable samples. They work without determination of absolute concentrations of analyte(s) and reference molecule(s). The result, correlated to the analyte to reference molecule ratio, is obtained in a single measurement.

### Description of the invention

The invention is based on novel methods of rapidly determining the relative concentration of analytes, wherein the results obtained are not a measure of absolute analyte concentrations but rather representative for the relative concentration of an analyte in relation to a reference molecule. The results of these measurements are correlated to the analyte to reference molecule ratio. This method for the detection of analyte to reference molecule-ratios is characterised in that while the sample passes through a device (a) a modified analyte-specific binding partner (masbp), which is bound to the reference molecule or a mimetic thereof (I) is released by the reference molecules in the sample and (II) a fraction of masbp forms analyte/masbp-complexes with analyte molecules present in the sample, (b) analyte not bound to masbp in (a (II)) is removed, (c) the complexes formed between analyte and masbp are measured, and, (d) the values measured in (c) are correlated to the values of an independently developed calibration curve for analyte to reference molecule ratios and thus analyte/reference ratios are assessed.

The modified analyte-specific binding partner (masbp) is a molecule combining at least two unique and essential features. On the one hand it is capable to bind to the analyte molecule, on the other hand it is modified to comprise one partner of a second and reference molecule-specific binding pair (e.g. an antibody to a reference molecule or the reference molecule recognised by this antibody). In one embodiment the reference molecule itself or an appropriate analogue or derivative (=mimetic) thereof is comprised in the masbp. In a second embodiment a specific binding partner to the reference molecule is part of the masbp.
The masbp is present in appropriate but limited amount. Release of masbp is dependent on the total amount of reference molecules migrating through the device. The volume required to release the masbp molecules is essentially the same volume out of which analyte molecules are determined. It is this "automatic" volume correction or volume control which brings about the great advantages of the invention. Once released, the masbp molecules migrate with the sample or assay liquid through the device. Analyte molecules contained in that part of the sample passing the device after masbp has been released can no longer be bound, because the released masbp migrates ahead of them. Figuratively phrased, analyte molecules contained in the sample liquid passing the device once masbp has been released come too late. This way the fraction of masbp molecules in complex form with analyte is always a correlate of the analyte to reference molecule ratio. Absolute concentrations of both reference molecule and analyte thus are not required to assess their concentration relative to each other.

In a preferred embodiment (monoclonal) antibodies are used to generate the masbp.

It is further preferred to additionally introduce a label or marker into masbp in order to render the masbp directly or indirectly detectable.

For procedures performed in analogy to the so-called sandwich (immuno-) assay principle it is further preferred to use a second analyte-specific binding partner in order to detect masbp-analyte-complexes. The binding of this second binding partner to the analyte is independent of whether the analyte is in free form or already bound to masbp. In a preferred embodiment this second analyte-specific binding partner is detectably labelled.

The invention also discloses devices enabling the determination of (an) analyte to reference molecule ratio(s) (A/RMR). For this determination the assessment of only one analyte-related signal is required. Such devices for measurement of analyte to reference molecule ratios, comprise at least (a) a first compartment I comprising (I) a solid support coated with a reference molecule or mimetic thereof, (II) a modified analyte-specific binding partner (masbp) capable of specifically binding to the analyte and being modified that it binds to said reference molecule or said :mimetic thereof said masbp being bound via said reference molecule or mimetic thereof to compartment I in a way that it can be released by reference molecules in the sample, and (b) a second compartment II, comprising means of removing any analyte not bound to said modified analyte-specific binding partner. Binding between analyte and masbp results in formation of an (immuno-) complex (=analyte/masbp-complex). This complex is not retained in compartment II. This basic design can be easily varied in that it comprises at least (a) a first compartment I comprising (I) a solid support coated with a binding partner for the reference molecule, (II) a modified analyte-specific binding partner capable of binding to the analyte and bei ng modified to carry the reference molecule or mimetics thereof, this modified analyte-specific binding partner being bound to the reference molecule of compartment I and capable of being released by the reference molecules of the sample, and (b) a second compartment II, comprising means of removing any analyte not bound to said analyte-specific modified binding partner.

The device according to the invention may be varied as appropriate to contain additional areas of special features and functions, like a detection zone, a safety zone or control means, areas for sample application and, where appropriate as well an area to which a detection reagent is applied.

It is preferred that the device in addition to compartment I and II comprises a detection area or zone.

It is further preferred that the device in addition to compartment I and II comprises a safety or control area, such area being designed to indicate that a sufficient amount of reference molecule has passed through the device and that release of masbp may be considered as completed.

Novel reagents to be used in the devices and according to the methods of the present invention are also disclosed. Such reagents are termed the modified analyte-specific binding partner (masbp). Two basic kinds of masbp are described. The first masbp is characterised in that said binding partner is modified to also bind to a reference molecule or a mimetic thereof. The second kind of masbp is characterised in that said binding partner comprises a second binding partner capable of specifically binding to the reference molecule or mimetics thereof. It is an important feature that the masbp binds to the reference molecule or a mimetic thereof in a reversible manner.

In a further improved embodiment the masbp is detectably labelled. Such masbp carrying a label are most appropriate in assay devices which work in analogy to the competitive immunoassay principle.

The reagents, methods and devices described can be used wherever analyte to reference molecule ratios are of diagnostic relevance.

These and other objects of the invention will be more fully understood when the following detailed description of the invention is read in conjunction with the accompanying drawings. The scope of the invention is further characterised in the claims.

### Detailed description of the invention

Some of the key features of the invention are depicted in the drawings and shall be described and discussed in more detail below.

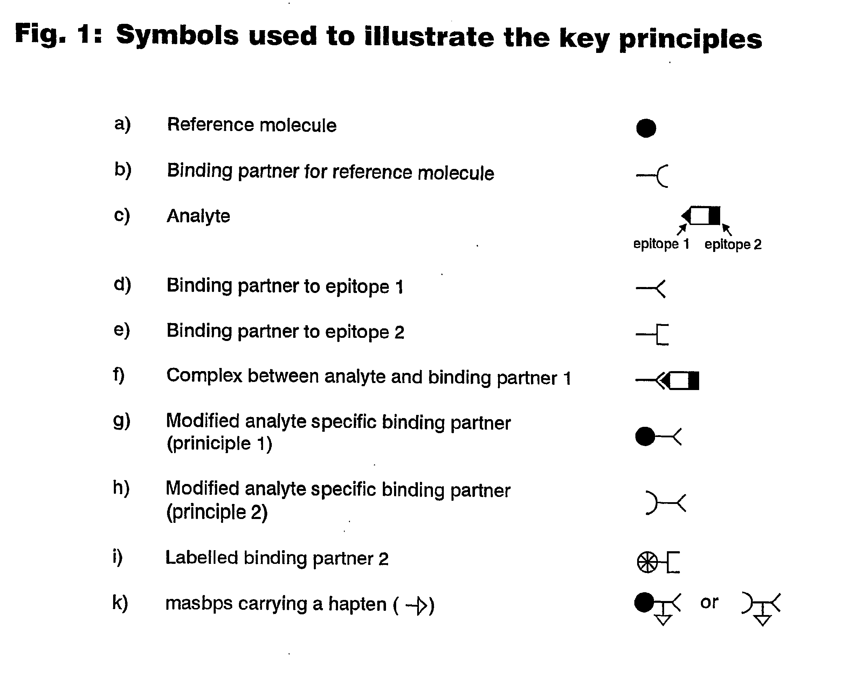

- Fig. 1: summarises the symbols used to illustrate the key principles of the present invention.
- Fig. 2-4: show different steps during the measurement according to key principle I, wherein the modified analyte-specific binding partner (masbp) carries the reference molecule and is bound to the solid phase of compartment I by aid of a binding partner specific for the reference molecule.

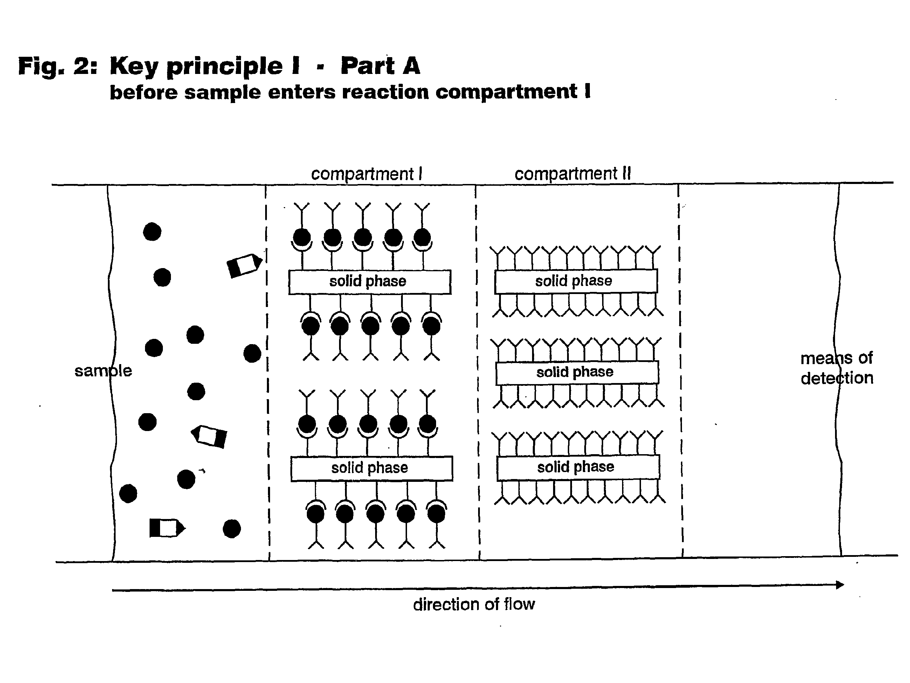

- Fig. 2: depicts the device according to key principle I before sample is added (allowed to enter (compartment I) of the device.

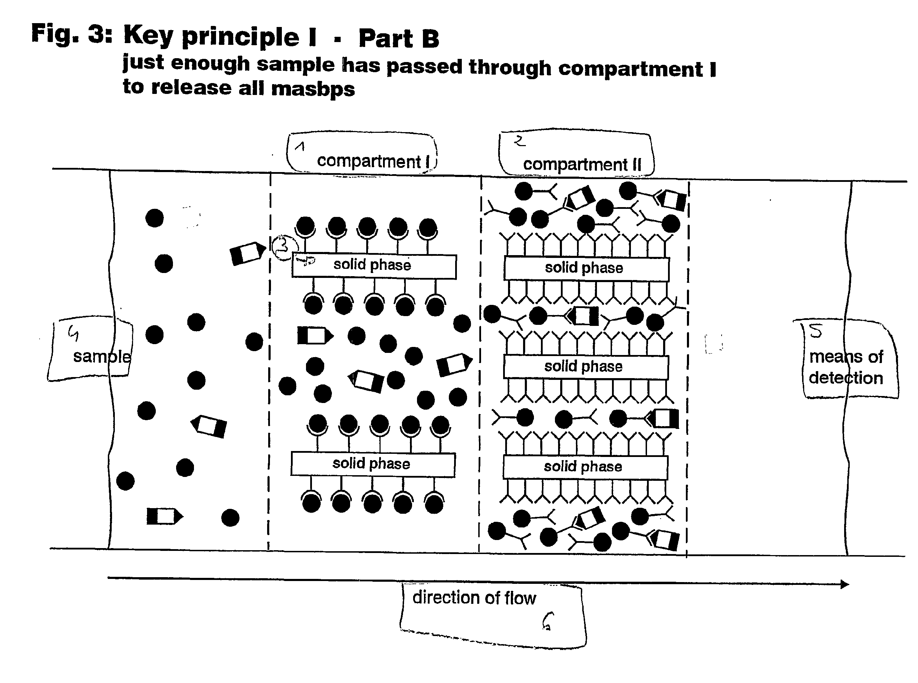

- Fig. 3.: shows the device according to key principle I during the measurement wherein just enough sample (just enough reference molecules) has passed through compartment I to release all masbps which are now found (either complexed with the analyte or w/o analyte) in compartment II.

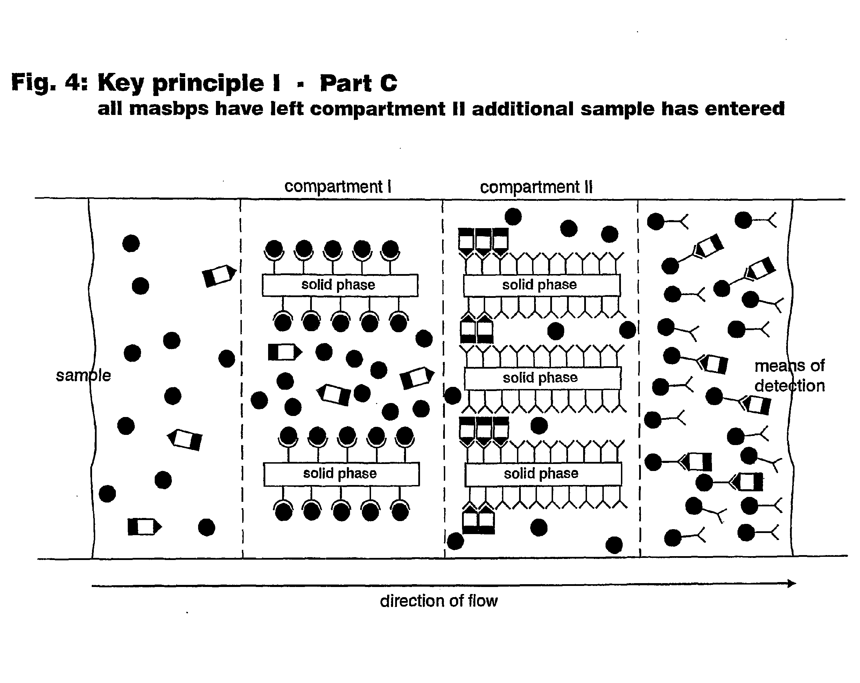

- Fig. 4: indicates that in the device according to key principle I any analyte not complexed to masbp will be retained in compartment II and that only analyte complexed with masbp has passed compartment II and is amenable to any appropriate mode of detection.

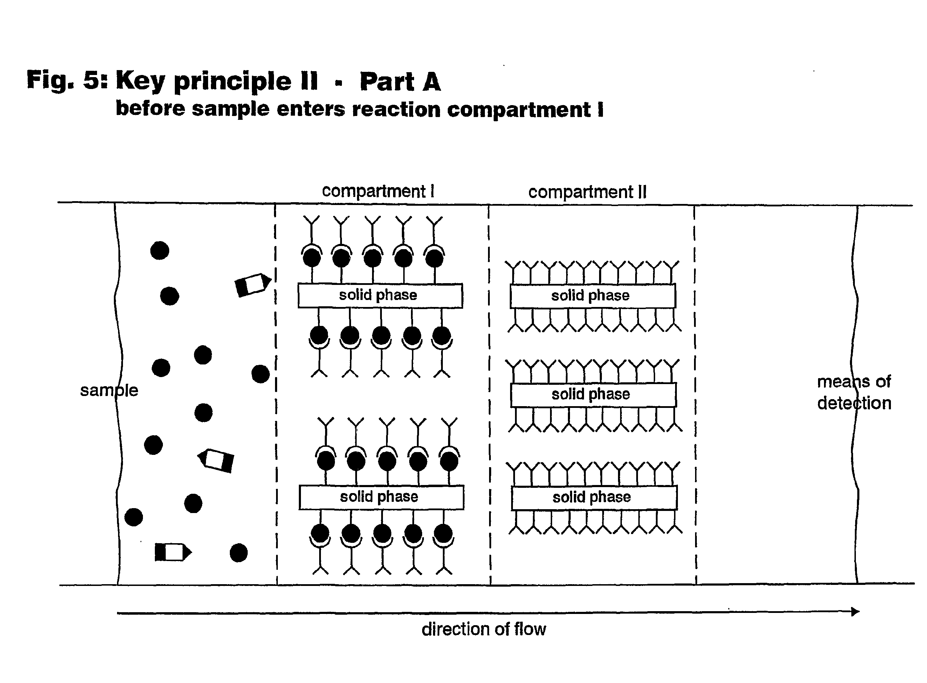

- Fig. 5-7: show different steps during the measurement according to key principle II, wherein the masbp has two binding specificities, one for the analyte and one for the reference molecule, and is bound to the solid phase of compartment I by aid of reference molecules or analogues thereof attached on the solid phase.
- Fig. 5: depicts the device of key principle II before sample is added/or not yet having entered the device (compartment I). All masbps are bound to the solid phase of compartment I.

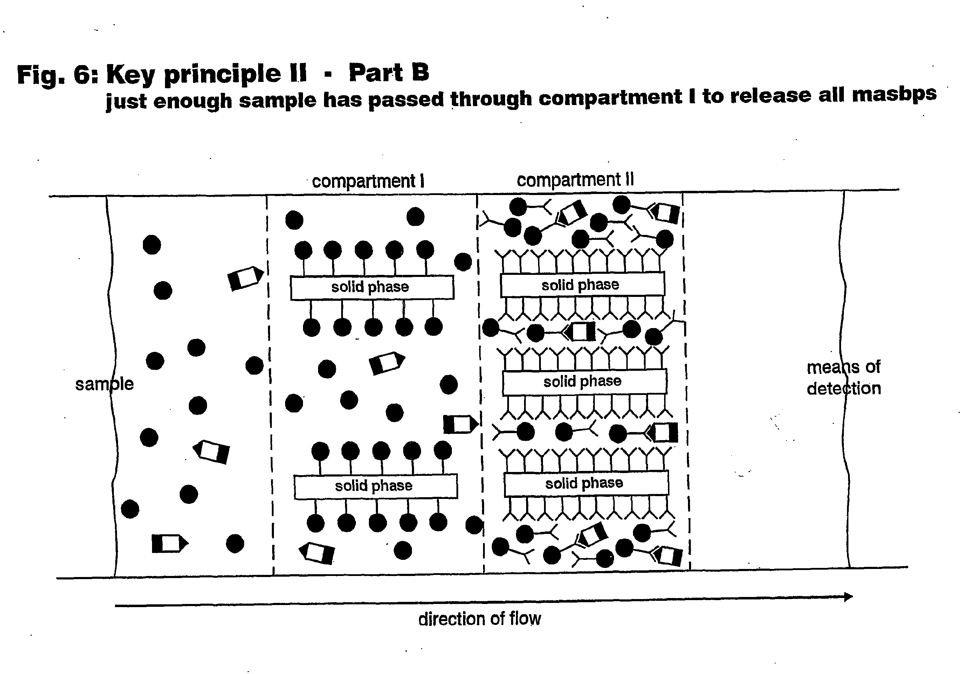

- Fig. 6: shows the device according to key principle 11 during the measurement wherein just enough sample (with just enough reference molecules) has passed through compartment I to release all masbps which are now found (either complexed with the analyte or w/o analyte) in compartment II.

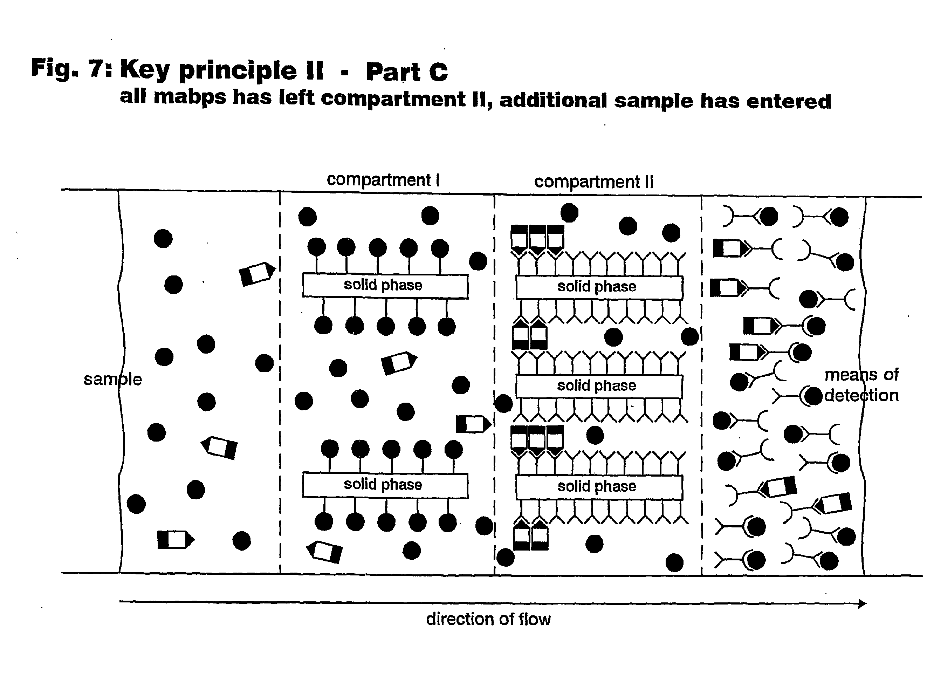

- Fig. 7: indicates that in the device according to key principle I any analyte not complexed to masbp will be retained in compartment II and that only analyte complexed with masbp has passed compartment II and is amenable to any appropriate mode of detection.

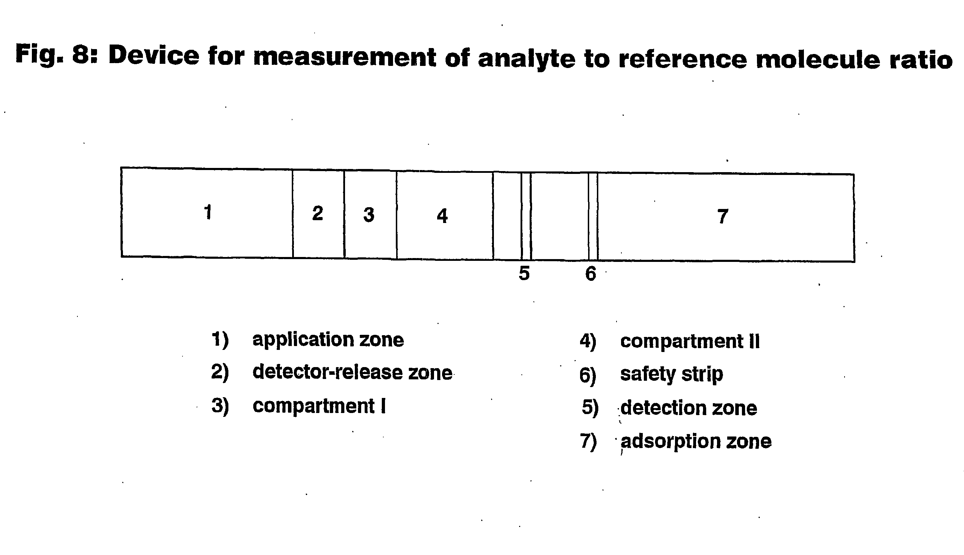

- Fig. 8: is a cartoon of a test strip (tow view), designed to measure the ratio of one analyte to one reference molecule based on a compartment I (3), a compartment II (4) and comprising additional areas which may be optionally included into such a device, e.g. a sample application area (1) a slow release area for labelled detection reagent (2), a detection zone (5) a safety means (6) in order to indicate that a sufficient amount of sample has passed through the device, and an absorption zone (7).

The procedures described in detail in textbooks like "Practice and Theory of Enzyme Immunoassays" by Tijssen, 1995, or "Immunochemical methods in the biological sciences: Enzymes and proteins", Mayer R.J., and Walker, J.H., Academic Press, New York, 1980; "Quantitative Enzyme Immunoassay", Engvall, E., and Pesce, A.J., Blackwell Scientific Publications, London (Scandinavian Journal of Immunology, 1978) are basic and general knowledge in this field.

The present invention is based on novel methods of rapidly determining the relative concentration of analytes, wherein the results obtained are not a measure of absolute analyte concentrations but rather representative for the relative concentration of an analyte in relation to a reference molecule. The results of these measurements are correlated to the analyte to reference molecule ratio. This method for the detection of analyte to reference molecule-ratios is characterised in that while the sample passes through a device (a) a modified analyte-specific binding reagent, which is bound to the reference molecule or a mimetic thereof (I) is released by the reference molecules in the sample and (II) a fraction of masbp forms analyte/masbp-complexes with analyte molecules present in the sample, (b) analyte not bound to masbp in a (II) is removed, (c) the complexes formed between analyte and masbp are measured, and, (d) the values measured in (c) are correlated to the values of an independently developed calibration curve for analyte to reference molecule ratios and thus analyte/reference ratios are assessed.

Test strip-like devices (also referred to as dry chemistry devices) have recently been developed in many different arrangements to cover various different clinical indications, sample sources and analytes. Especially for urinalysis there have been many improvements and it is not possible neither necessary to cover this field in great detail here. The skilled artisan has no problem to find the information necessary in the literature pertaining to this field in order to set up such devices. The use of reagent-impregnated test strips in specific binding assays, such as immunoassays is described in the relevant patent literature, e.g. in GB 1589234, EP 225054, EP 183 442, EP 186 799, EP 212 603 and EP 291 194. In the procedures disclosed there, a sample is applied to one part of the device and is allowed to permeate through the other parts and material(s) of the device. The sample itself or a "solvent", in most cases water or a water based buffer will elute all the reagents present in the device and necessary to obtain specific binding reactions. The complexes between analyte and binding reagent are usually bound or "trapped" in an detection zone and detection is performed by aid of labelled reagents which have already been included into this device or applied thereto subsequently.

The methods and devices according to the present invention require that at least two different compartments adapted for unidirectional flow of liquid are used. These two compartments most preferably are in direct contact thus allowing for lateral and unidirectional flow of liquid through these compartments.

In compartment I a so-called modified analyte-specific binding partner (masbp) is attached to the solid support of this compartment. Such attachment is mediated by the reference molecule or a mimetic thereof and a specific binding partner to this reference molecule.

In one embodiment the masbp carries the reference molecule or an analogue thereof and is bound to the solid support by action of a reference molecule specific binding partner (rmsbp) (cf. key principle I; Fig: 2-4). The rmsbp is attached to the solid support of compartment I.

In a further embodiment (cf. key principle II Fig: 5-7) the masbp is characterised in that it carries binding partners for both the analyte as well as the reference molecule. In this embodiment the masbp is also attached to support via the rmsbp. In the second embodiment the reference molecules or analogues thereof are fixed to the solid support and the rmsbp is bo und via reference molecules or appropriate mimetics thereof to compartment I.

Like in the first embodiment the reference molecules in the sample are capable of releasing masbp from compartment I. The binding between reference molecule and the rmsbp is reversible and during analysis reversed by reference molecules contained in the sample.

The appropriate amount of reference molecules or its binding partner which has to be bound in compartment I can be determined by standard immunological titration procedures. The amount of masbp is varied as required, especially depending on the reference molecule and its binding partner used. During analysis the masbp is released from compartment I, because the reference molecules of the sample compete for the corresponding binding sites on the masbp.

It has to be safeguarded that enough reference molecule passes through the device to completely release the masbp of compartment I. In some cases, e.g. with rather concentrated samples under investigation, there is enough reference molecules in such samples to ensure full release of the masbp of compartment I. With such samples, e.g., the sample application area of the device may be placed into the sample and results read, once the liquid flow has reached the liquid sink or absorption zone (Fig.: 8 (7)). With highly variable samples, which as well may be rather dilute, it is highly desirable to know that, or when enough sample has passed the device. This is encompassed by additionally including a safety or control area into the device, which indicates that enough reference molecules have passed through the assay system.

The second compartment (cf. compartment II in Figs 2-8) contains a means for retaining any analyte not complexed to masbp, Whereas many different ways can be designed to ensure that compartment II works as required, the most easy and most preferred design is to use the analyte-specific binding partner of compartment I and fix it to the solid support of compartment II. When the assay is performed and sample liquid passes through compartment II, compartment II works as a means for retaining any analyte not complexed to the masbp. Importantly, analyte complexed (preferably immunologically bound) to the masbp will not be retained and pass through compartment II, while the sample (reaction) fluid passes through compartment II. With other words, only those analyte molecules complexed with the masbp can pass compartment II and are amenable to any appropriate means of detection thereafter.

As mentioned, reference molecules in the sample compete for binding sites of the reference molecule-masbp-complex in compartment I and bring about release of the masbp. Rate of release of masbp from compartment I is correlated to the concentration of reference molecule in the sample, i.e., the higher the concentration of the reference molecule in a sample the less total volume of sample is required to release the masbp of compartment I, the more dilute the sample, the higher the volume required. Once released masbp migrate with the sample liquid through the device.

The analyte molecules in the sample as well migrate with the sample liquid through the device. Those analyte molecules reaching the masbp before they are released or coming in contact with released masbp during their migration will form an analyte/masbp-complex (= complex between analyte and masbp). Only those analyte molecules contained in that volume of sample required to release the masbp of compartment I can react to form such a complex. With other words, it is only the analyte molecules contained in the sample volume corresponding to the amount of reference molecules required for release of the masbp which can bind to masbp. Once all masbp is released and has entered compartment II no more analyte can bind to the masbp, because any additional analyte will be retained by the means of compartment II.

The first step of the inventive method, characterised by the release of masbp due to the reference molecules in the sample and the formation of analyte/masbp-complexes for a fraction of the masbp released is over a wide range not dependent on the concentration or dilution of the sample. The limiting factor is the total amount of reference molecules. This aspect is key to the understanding of the invention. It shall be exemplified by means of theoretical examples. 1000 masbp are bound in compartment I. Sample A contains 1000 reference molecules (rms) and 100 analyte molecules (ams) in 100 µl; sample B contains the same 1000 rms and 100 ams but in 10µl, whereas sample C contains 1000 rms and 300 ams in 50 µl, respectively. (For ease of explanation the volume of the compartments is considered to be negligibly low). For sample A, it takes 100 µl of sample to release all 1000 masbp, since 100 analyte molecules are present in that volume 10% of the masbp released will be complexed to analyte. This percentage (10%) will be the same for sample B despite the fact that the sample had the tenfold absolute concentration of both analyte and reference molecules. For sample C, 300 analyte molecules (corresponding to 1000 reference molecules in 50 µl will form an analyte/masbp-complex, i.e. 30% of the masbp will be complexed when entering compartment II. For all three samples the analyte-dependent signal as measured thereafter according to standard procedures will correctly reflect the ratio of analyte to reference molecule. As exemplified, all these ratios are assessed independently of knowing the absolute concentrations of both the analyte and the reference molecule in the sample.

The fraction (ratio) of masbp being complexed to the analyte correlates to the ratio of analyte to reference molecules in the sample. It is over a wide range independent of the sample volume or the concentration/dilution of the sample.

The analyte/masbp-complex can be detected by any appropriate means once it has passed through compartment II.

The term analyte as used herein covers all biological molecules of diagnostic interest capable of binding to a specific binding partner. Members of binding partners, binding reagents or binding pairs are well-known to the experts in the field. Specific reference shall be made to the binding pairs most widely used such as nucleic acid/nucleic acid; co-enzyme/enzyme; biotin/(strept-) avidin; sugar/lectin; antigen/antibody; hapten/antibody (cf., e.g. Tijssen, 1995, supra).

The reference molecule in the sense of the present invention shall mean any substance which may be used to normalise an analyte measured in a sample to it. Any molecule which may function to correct for variations in the concentration or dilution, respectively, of a sample may be used. Appropriate examples are e.g., amylase for saliva, some testosterone derivatives, collagen degradation products and creatinine for urine. Selected examples of sources for samples as well as reference molecules appropriate for such samples will be discussed below.

The expert in the field will appreciate that in special settings and for a number of reasons it may be worth while to use analogues of the reference molecule as a partner of the masbp in compartment I. Especially analogues having a reduced (2-, 3-, 5-,10-,up to 50-fold lower) affinity for binding to the masbp will be considered, whenever fast release of masbp is req uired.
In other settings, where the concentration of reference molecules, as compared to analyte(s) is high. The expert will select analogues having increased affinity to the rmsbp. Such increase in affinity may be up to 50-fold. In most cases affinity-increases up to 2-,3-,5-or 10 fold in will be considered sufficient.

Procedures for making such analyte analogues for peptides and/or proteins are described in the relevant (patent) literature, e.g. in WO 91/13909 or WO 95/20764.

The binding partner for the reference molecule (rmsbp) shall bind the reference molecule (or its analogue) in a manner, which is reversible. Such reversion (bringing about the release of the masbp) is triggered by reference molecules passing through compartment I.

The modified analyte-specific binding partner (masbp), according to the embodiments of the present invention, can be modified in at least two quite different ways.

According to key principle I, as illustrated in Figs. 2-4, the reference molecule (or analogue) is attached (or linked) to the (first) analyte-specific binding partner. Such attachment preferably comprises a covalent chemical linkage or makes use of binding partners like biotin and streptavidin, having affinity constants of at least 10⁻¹² M/I.

According to key principle II, as exemplified in Figs. 5-7, a binding partner for the reference molecule is attached to the analyte-specific (first) binding partner by standard procedures, e.g. as mentioned above. In addition .to these conventional ways of attachment, chimaeric antibodies as well as appropriate nucleic acid constructs are also quite appropriate tools.

The second unique step of the inventive method is the fact that analyte not bound to masbp (in the first step of the inventive method, e.g., while passing compartment I) is removed. This removal can be accomplished by any appropriate binding reagent which does not bind significantly (not to an extend which would interfere with the procedure) to a complex between analyte and masbp but on the other hand is capable of removing any analyte not part of such a complex. Preferably the binding reagent of compartment II is present in large amounts. It is thus preferred that the binding capacity for analyte of compartment II is five times, more preferred ten times, more preferred twenty times, more preferred fifty times or more preferably more than fifty times the analyte binding capacity of the masbp bound in compartment I. The high concentration of the means to remove any non-bound analyte in compartment II ensures that no "overflow" of analyte is likely to occur. Such overflow would otherwise disturb the measurement.

Whereas the binding between the partners of the reference molecule binding pair must be reversible, the binding between masbp and analyte preferably is as strong as possible with an affinity preferably above 10'' M/I; more preferred above 10⁻⁸ M/l, more preferred above 10⁻⁹ M/I e.g., characterised by rather a slow dissociation of these two molecules.

The firm attachment or linking, e.g., used for coating the solid phase of compartment I or II with e.g. the rmsbp, the reference molecule or the means to remove excess analyte, respectively, is essentially irreversible.

The solid phase of compartments I and, or II may consist of any appropriate material. Preferred are glass, latex or plastic beads filled into an appropriate column even more preferred are membranous materials allowing for migration or lateral flow of materials through the compartments of the invention. Membranes of bibulous as well as non-bibulous nature are most preferred to carry out the present invention.

In addition to the two essential compartments as described above one or several additional compartment(s) may be part of the device. Design and functions of these additional compartments and the chemical or immunological procedures taking place there may vary depending on the analyte(s) investigated as well as on the source of sample and the reference molecule used.

With some highly variable sample sources it may be necessary to include a safety zone (cf. 6 in Figure 8). This zone is designed to indicate any "overflow" of reference molecule. It can be designed in various modes. Most preferred the safety zone comprises a labelled reagent capable of being released by excess reference molecule, most preferred by mechanisms comparable to the ones used in compartment I. Release of even only a small part of such a labelled reagent implies and ensures that enough sample (reference molecule in excess) has passed through the device and that release of masbp is complete. Most preferred are designs which can be evaluated by eye.

Detection of the fraction of masbp either bound to analyte or still free (once enough sample has passed through compartments I and II of the device) can be performed by a variety of procedures known to the skilled artisan. A lot of important methods pertinent to the field are described in textbooks. E.g, the procedures described in detail in textbooks like "Practice and Theory of Enzyme Immunoassays" by Tijssen, 1995, or "Immunochemical methods in the biological sciences: Enzymes and proteins", Mayer R.J., and Walker, J.H., Academic Press, New York, 1980; "Quantitative Enzyme Immunoassay", Engvall, E., and Pesce, A.J., Blackwell Scientific Publications, London (Scandinavian Journal of Immunology, 1978) are basic and general knowledge in this field.

A second analyte-specific binding partner which is detectable or labelled may be used. Of course the binding of this labelled (second) analyte-specific binding partner (lasbp) must not interfere with the binding of analyte to masbp in compartment I. The lasbp may be part of the device, e.g., contained in an application zone or in a special release zone (cf. 1 or 2 of Fig.8).

The laspb may be incorporated into the device such that slow release is ensured as long as sample (analyte) enters into the device. These ways, the vast majority of analytes will form a complex with the lasbp before entering into compartment I. Alternative designs, wherein the lasbp is contained in a different zone of the device or is added subsequently are as well possible. The lasbp may alternatively be added to the sample before this is brought into contact with the test device of the invention.

Appropriate labels are well known to the expert in the field. Only a few representative examples of such labels, like enzymes, coloured latex particles, fluorescent or chemiluminescent labels, or colloidal metals, like colloidal gold shall be specifically mentioned. Coloured latex particles and colloidal gold particles are preferred labels.
Labelling is performed according to standard procedures, e.g. as described in Tijssen, 1995, supra, or J. Beesley, "Colloidal gold: A New Perspective for Cytochemical marking", Microscopy handbooks 17, Oxford University Press, 1989.
Detection of analyte/masbp-complexes is performed according to standard procedures. In one preferred embodiment a special detection zone is part of the device and is used to carry out the measurement of analyte molecules contained in these complexes. Most preferably the detection zone (5 in Fig. 8) is coated with a receptor specific for the first masbp. The analyte/masbp-complex is then detected/measured e.g. by means of a second analyte specific binding partner (not binding to the receptor for the masbp) which is detectable or labelled. In case both analyte-specific binding partners are monoclonal antibodies from the same species the skilled artisan will modify the masbp to additionally contain a unique binding pair member, e.g. the antibody of the masbp will be selected to represent a different subclass of immunoglobulin, as compared to the lasbp or modified to comprise a hapten, e.g. dig(it)oxin or biotin.

In other cases, e.g. with very small analytes (where binding of two different binding partners to the analyte is not possible) the skilled artisan will make use of competitive assay designs.

Such assays preferably make use of labelled analyte derivatives which will bind to the unoccupied binding sites of the masbp and use such derivatives in order to determine analyte to reference molecule ratios.

Alternatively and even more preferred labelled masbp is used to assess those masbp not part of a complex with analyte.

The results of measurements according to the present invention are preferably assessed by eye or by any appropriate means or device for measuring the label employed. Such assessment is greatly facilitated if labels are present rather concentrated e.g., when using a detection area or zone in form of a line on a teat strip device. The results obtained are correlated to the analyte to reference molecule ratio in the sample investigated, and preferably are deduced or calculated from a calibration or a standard curves for the analyte to reference molecule ratio under assay.

Methods to establish standard or calibration curves in general or for analyte to reference ratios as discussed here, are in-numerous. Standard values and standard curves depend largely on the assay procedures applied and to a great deal on the procedures used to standardise or calibrate these assays. Specific assays e.g., based on the principles of competitive or sandwich immunoassays are available or can be designed to establish concentrations of the analyte(s) and the reference molecule individually. Such individual values can be used to establish calibration or standard curves for analyte to reference molecule ratio(s). Unknown samples can be analysed and analyte to reference molecule ratios extrapolated from previously or parallel established standard curves. In most cases such standard curves will be used to standardise the devices of the present invention. Such standardisation usually is performed by the manufacturer of a device. Results measured with samples are correlated to such standardisation or calibration data and the ratio of analyte to reference molecule thus deduced. It is preferred that data are calculated from standard curves which are independently developed and used to calibrate the inventive device.

As mentioned above the reagents, methods, and devices of the present invention can be applied with great advantage to variable samples, i.e., samples, like the ones discussed below. Detailed description for some selected sources of samples.

### Saliva as Source of Analytes:

In EP 0 753 148 B1 saliva is used as source of analytes. Reference is given there to a lot of patents dealing with problems associated with saliva as a sample and to appropriate solutions to tackle some of these problems. Probably the most serious problem, when using saliva as a sample, is the fact, that saliva is not a uniform sample. Rather different concentration/dilution of this sample and therefore also of analytes contained therein will regularly be encountered. Various patents have sought to define means to correct for the relative dilution/concentration of analytes in saliva samples. US 5,534,502 and PCT/WO 93/11434 describe devices for determining that a (saliva) sample collected for diagnostic purposes is in fact saliva. Both are based on demonstrating the presence of amylase activity in the sample.

EP 0753 148 B1 further improves the testing of analytes in saliva by defining an amylase cut-off-value. By defining such an amylase cut-off it is ensured that at least the minimum amount of sample required for a meaningful analysis is present. However, it will be obvious to the expert in the field that a device as described in EP 0 753 148 B1 only can produce qualitative, yes-or-no types of results.

According to the present invention analyte to reference values can be obtained for samples comprising saliva. Of the lot of different embodiments possible only one device and one procedure will be described in detail. Amylase is referred to as the reference molecule, though any other molecule present in saliva and being appropriate for use as a reference molecule, alike amylase, may also be chosen.

In a preferred embodiment antibodies to amylase will be fixed in compartment I in an appropriate concentration. Appropriate concentrations can easily be determined by the skilled artisan using standard titration techniques routinely used in the development of immunoassays. Also present in compartment I and bound via the anti-amylase-antibody will be the modified analyte-specific binding partner (masbp).

In a preferred embodiment the masbp is an antibody specifically reactive to the analyte of interest and conjugated with amylase or a fragment or derivative thereof being capable of binding to anti-amylase antibody and being bound in such a manner that amylase in the sample causes release of the masbp. Enzymatic properties of amylase may also be used for binding and release of the masbp.

Compartment II in a preferred embodiment contains the analyte specific binding partner (asbp) used for producing the masbp of compartment I. However, the asbp of this compartment is fixed to the solid phase. In a preferred embodiment it will be present in amounts high enough to retain any analyte not bound (complexed) to masbp in compartment I. It will be obvious to the skilled artisan that analyte-specific binding partners other than the one used as the starting material for masbp can be used in compartment II, provided that the masbp/analyte-complex is not retained and that free (non-complexed) analyte is retained.

Detection of the analyte/masbp-complex can be performed by any standard diagnostic means. Such means are well-known to the skilled artisan.

Analyte in the case of saliva may mean any molecule of diagnostic interest, e.g. drugs like cotinine, cocaine, (meth)amphetamine, antibodies to infectious agents like HCV or HIV or other viruses bacteria or fungi, as well as antigens, like hormones, viral proteins, e.g., hepatitis B surface antigen, or collagen degradation products.

It is expected that by providing the diagnostic means of the present invention the list of analytes which can be investigated in order to obtain clinically relevant information from saliva will largely increase due to the higher relevance, comparability and significance of dilution-corrected analyte measurements made possible by the reference molecule correction of the present invention.

### Exudate fluid

Recently methods have been developed and means have been designed to collect so-called exudate fluid (US 6 048 337). Exudate fluid is quite variable and measures for standardisation have to be taken. One of the procedures used is to use a salt contained in the exudate fluid, like sodium or potassium to normalise for exudate concentration.

Exudate is also known to contain small peptides, e.g. peptides derived from collagen degradation. It is envisaged that such and similar peptides or other small molecules will be used to directly determine analyte to reference molecule ratios in exudate fluids in methods performed according to the present invention.

### Urine as a sample

Most diagnostic procedures today are based on the analysis of serum, blood or plasma. Rather few parameters are diagnosed from urine despite the fact that it can be most easily obtained.

The major disadvantage of urine resides in the fact that it does not represent a uniform more or less constant sample source. Instead, the concentration of analytes in urine is largely dependent on the urinary excretion rate which in turn is dependent on a lot of parameters of different origin, like fluid intake, physical activities, day-time, stress etc.. Where only qualitative results are required, e.g. for proving the presence or absence certain drugs, urine is the sample of choice.

Whenever an analyte shall be determined in a manner not dependent an urine dilution or excretion rate, special and time-consuming measures have to be taken. Either 24 hour-collections of urine have to be considered or the analyte measurement has to be corrected for sample dilution by aid of a reference molecule, e.g. like creatinine.

Analytes of interest in urine are especially those molecules, small enough to pass the healthy kidney, e.g. hormones, drugs and their metabolites or degradation products of larger proteins. In cases of kidney damages also large proteins, like albumin will be found in urine and can provide clinically relevant information.

During the course of diabetes a significant percentage of patients will suffer from what is called "renal involvement". This term is used to indicate that disease processes eventually lead to a decreased renal function. Whereas only rather small molecules in the 5-10 kd-range and below, are able to pass through a healthy kidney an affected kidney of diabetics is no longer able to completely retain serum albumin, which has a molecular weight of about 60 kd. Urine of diabetics with kidney damages thus contains, e.g., serum albumin in addition to the small molecules found in urine of non-diabetics. Severe kidney damage, accompanied by large amounts of serum albumin in urine, may be detected by qualitative or semi-quantitative measurement of serum albumin from urine. In cases where albumin is only moderately increased, e.g. in the early stages of renal complications, values for serum albumin which are corrected for concentration/dilution of the sample e.g., by making reference to creatinine do compensate for the variability caused by urine.

In a preferred embodiment the analyte will be serum albumin, which has leaked into urine due to kidney damage. It is expected that kidney damage will be better monitored by using the method of the invention as compared to semi-quantitative non-corrected measurements as currently applied in the art.

Other examples of analytes frequently measured from urine are female hormones and collagen degradation products (CDPs). Measurement of both these types of analytes in relation to reference molecules is discussed herein after.

Pregnancy can be easily diagnosed from urine by qualitative measurement of human chorionic gonadotropin (HCG) which is only present during pregnancy. This is possible because the mere presence of this hormone is indicative for pregnancy and thus there is no need to know the corresponding concentration of HCG in blood or to compensate for volume/ concentration effects of the urine used.

Variables of the ovulation cycle are also measured from urine. In order to do so, hormones, like estradiol or a metabolite thereof (estrone-3-glucuronide = E3G) are quantified. It is well known that estradiol increases several days in advance of the ovulation. However, a variable which will interfere with the comparability and utility of such measurements is "volume" or concentration of the body fluid used. This is particularly important and significant in relation to urine, which represents the most commonly chosen and most convenient source of sample for assessing the ovulation cycle.

The degree of fluid intake, kidney function, physical activities, stress, etc. all have very significant influence on actual volume and dilution/concentration (dilution or concentration) of the urine excreted. The apparent concentration found in urine therefore may not be a true reflection of the amount produced in the body at this point in time. Despite these problems, urine represents a very convenient source of sample. It can be easily collected, if required several times a day. Sampling for blood, plasma or serum is much more complicated and time-consuming and to some extend invasive as well.

WO 95/13543 discloses and recommends to use testosterone-17β-glucuronide (T17G) as a reference molecule or "volume corrector" when measuring E3G to assess the ovulation cycle. This is preferred as compared to the use of creatinine, because both E3G and T17G are found in urine in a comparable range of concentration, whereas creatinine is much more abundant. It might further be of advantage that both molecules are hormones and do not depend on muscle or body mass and physiological activity as is true to some extend for creatinine.

Antibodies to E3G and T17G which are well-known in the art as well as convention al ways to synthesise T17G- and E3G-conjugates (see especially WO 95/13543) can be used to carry out the present invention. In a preferred embodiment an antibody to T17G is bound to compartment I. T17G is coupled to the antibody specific for E3G. This conjugate represents the masbp according to this embodiment. Compartment II is coated with an antibody specific for E3G which does not bind to E3G already bound (complexed) to masbp.

There are various possibilities which are known to the skilled artisan, in which the masbp to analyte complexes can be detected. Most preferred are procedures in which the amount of masbp not complexed with analyte (binding site not occupied by E3G) are detected. Preferably in this test design the antibody specific for E3G will not only be modified to carry T17G (= masbp), but rather this masbp will as well be directly labelled or be further modified to carry a hapten to facilitate indirect labelling. Directly labelled refers to any kind of label which is attached or bound to the masbp. Indirectly labelled e.g. by a hapten refers to binding pairs like biotin/streptavidin or hapten/anti-hapten antibody. In the latter case a binding partner of this binding pair is labelled, e.g., labelled streptavidin binding to biotinylated masbp is used.

The above procedure is given according to key principle I, i.e., the reference molecule (T17G) is attached to the analyte-specific binding partner (antibody to E3G). Of course the invention may also be carried out according to key principle II, making use of the disclosure as given above. In addition the skilled artisan will appreciate that diagnostically relevant data may also be obtained when E3G and T17G are used as reference molecule and analyte, respectively.

Results obtained in the above measurements of hormone ratios are read by eye or optically measured and correlated to previously and independently established standard curves for these ratios. The measurement can be used to follow the ovulation cycle and to correctly predict ovulation.

Whereas the above disclosed procedures make use of the ratio between E3G and T17G, the ratio of E3G to creatinine also may be used and analysed according to the present invention.

There are numerous ways to design devices for measurement of the E3G-to-T17G ratio. In a preferred embodiment the device comprises a compartment I to which an antibody to T17G is "fixed" and the masbp bound via the anti-T17G antibody, and a compartment II comprising a binding partner for E3G but not reactive with E3G bound to masbp. Further embodiments may additionally comprise a detection zone, and in addition to that also a safety zone. It is obvious to the skilled artisan that chemicals and reagents which are of use in these procedures can be used as required without departing from the spirit of the invention.

The field of osteoporosis and its diagnosis from urinary samples may be considered to be one of the most advanced - though still a quite controversy area - of "quantitative" diagnosis using urine as a sample. In recent years a lot of different attempts have been undertaken to diagnose osteoporosis through analysis of biomarkers from urinary samples. The field of osteoporosis as well as the tremendous advantages of the present invention shall be described in some detail.

Diseases of bone, among these osteoporosis, are becoming an increasing burden to society. The total cost in the USA in 1992 of osteoporosis related injuries alone is estimated to be at least USD 10 billion (Riggs, L., New England Journal of Medicine, 327:620-627 (1992)).

Osteoporosis as well as a number of other diseases of bone is characterised by an increased rate of bone loss when compared to the rate of loss in a healthy population. Biochemical markers of bone metabolism have been shown to be highly correlated to the future fracture risk (Christiansen et al., Prediction of future fracture risk. In: Christiansen et al., eds., Proceedings 1993, Fourth International Symposium on Osteoporosis, Hong Kong, Osteopress Aps 1993; pp. 52-54) or indicative for successful treatment (Bjamason, N. H., Christiansen C., Bone 26(6), p. 553-560, 2000). Therefore assessment of biochemical markers of bone turnover is an important tool to aid the diagnosis of diseases which result in changes of bone metabolism, no matter whether this results in increased bone formation or bone resorption.

Due to the fact that osteoporosis is clearly preventable but only partially treatable, the early detection of osteoporosis is crucial if bone mineral content is to be preserved in menopausal adults and bone deterioration is to be prevented early on. Studies have shown that the rate of bone loss after menopause is frequently increased as compared to the rate of loss in pre-menopausal women. Many women in the first years after menopause are losing bone at a rate of greater than 3% and up to 7% per year. Further, in the majority of patients presenting with osteoporosis, 20%-40% of Bone Mineral Content has already been lost before diagnosis is made.

A variety of technically sophisticated methods have been developed to assist in predicting the likelihood of bone fractures, such as bone densitometry and quantitative ultrasound procedures. Both of which represent a highly specialised means for measuring bone mineral density (BMD or bone mineral content (BMC). These densitometric measurements provide a static picture and do not give any clue regarding the metabolic events going on in bone tissue. Only the repeated measurements after many months can be used to assess a significant change in BMD. Hence, when these tests are performed, in the majority of cases they only confirm whether a patient has lost significant quantities of bone mineral content already or not.

Such densitometric approaches are of limited use in actually diagnosing those (e.g., peri-menopausal adults who are likely to become osteoporotic, or are at risk to develop osteoporosis. In addition, these procedures also are quite expensive and not always disembursed by public health care providers, e.g. in Germany.

For a more successful detection of the onset of osteoporosis the use of serum or urinary concentrations of key biochemical markers has been suggested e.g. by Delmas, P. D. et al., Journal of Bone and Mineral Research (1986), 1: 333-337.

Most of the more recent approaches to diagnose osteoporosis from urine are subject to patent applications and several of them shall be mentioned in some detail.

PCT/WO 96/04544 is entitled "Urinary Test Strip for Determining Calcium Loss". It describes in great detail the problems encountered when using urine as a sample.
If dietary effects are negligible, the amount of calcium excreted into urine is directly related to bone turn-over. And, under the proviso, that no other factors, like diet, contribute significantly to calcium excretion, total urinary calcium is related to bone turnover, bone resorption or bone loss. However, either 24h urine samples have to be collected and the amount of calcium excreted per 24 hours has to be determined or, its excretion has to be matched against the known constant excretion product of urinary creatinine. The ratio between calcium and creatinine is said to be applicable to the detection of osteoporosis.

PCT/WO 96/04544 teaches the use of test strips comprising means for two independent measurements, one for measurement for calcium and one for measurement of creatinine.
Both molecules are measured independently based on the calcium- and on the creatinine-specific colour developed in the respective area of the test strip. Colours generated can be read visually or by photometric devices and are used as basis for the calculation of the calcium to creatinine ratio. The improvement of such a device being that both independent reactions are performed on the same test strip, as compared to the conventional approach of measuring both molecules on clinical-chemical analysers and thereafter calculating the ratios.

Whereas PCT/WO 96/04554 measures an inorganic component of bone (calcium) the most advanced technologies to detect osteoporotic conditions are based on the measurement of organic material released from bone during bone turn-over.

About 90% of the organic material found in the extra-cellular matrix of bone is type I collagen. During (physiological or pathological) bone turn-over osteoclasts resorp bone matrix, thereby releasing collagen degradation products (CDPs) and forming so-called resorption lacunes. Osteoblast or osteoblast (precursors) attach to the resorption sites and new bone matrix is formed over time. In healthy adults the resorption and the formation of bone are in equilibrium and bone mass stays constant. Osteoporosis, especially postmenopausal osteoporosis, especially in the first ten to fifteen years after menopause is characterised by a negative bone-balance, i.e. more bone is resorped that formed. This results in the above mentioned loss of bone and eventually in the disease called osteoporosis.

Several recent attempts to diagnose osteoporosis and to monitor bone turnover have focused on the measurement of special collagen degradation products (CDPs). In the past amino acid derivatives, typical for collagen, like hydroxyproline or hydroxylysine have been used.

Other methods relying on special cross-linking structures of type I collagen have been described, e.g. in US 5,700,693.

Nowadays much focus clearly is on short peptides, which are released upon or after the degradative action of osteoclasts. These collagen degradation products comprise cross-linked collagen telo-peptides derived from the N-terminal (NTX) and C-terminal (CTX) part of the collagen molecule.

In EP-B-0 394 296, a method for measuring bone resorption is described, based on immunological reagents with specificity to both, the cross-linking structure itself (a pyridinium cross-link) and to the peptide sequence attached to this cross-link. A product based on this approach is FDA-registered and called Osteomark™.

Others (Roche Diagnostics in EP-B-0 711 415, Osteometer in PCT/WO 91/13909) have used synthetic peptides as immunogens and found that antibodies to collagen peptides, which do not depend on the presence or absence of a (pyridinium) cross-link provide also very valuable tools in order to measure CDPs. Whereas Osteomark™ measures amino- or N-terminal CDPs (NTX) the alternative product CrossLaps™ from Osteometer measures carboxy-terminal or C-terminal CPDs (CTX).

It was not until quite recently that it has been discovered that some of the C-terminal CDPs contain a fairly unusual β-aspartic amino acid linkage (Bonde et al., PCT/WO 96/12193; Fledelius et al., The Journal of Biological Chemistry (1997), 272 (15): 9755-9763). Such β-linkage is typical for collagen released from "old" bone. Newly formed (and degraded) collagen on the other hand contains the normal linkage of aspartic acid to glycine in the so-called 8AA-peptide of CrossLaps. The skilled artisan will find in these patent families the technical advice required for production of specific antibodies as well as for obtaining naturally occurring or synthetically produced analytes or analyte analogues. Especially the synthetic peptides representing CDP-epitopes as disclosed in EP-B-711 415, WO 95/08115, PCT/WO 96/36645 and PCT/WO 98/26286 are useful to generate antibodies which may be used in a device or procedures according to the present invention.

Information of a more general nature on how to synthesise, screen for and to use synthetic peptides as well as so-called mimetics of peptides and other epitopes can be found in WO 91/13909 + WO 95/20 764. Such mimetics or analogues of analytes or the reference molecule may be used to modify (as required) the binding and release properties of the masbp by the reference molecule in the sample.

All the above methods for analysing bone metabolism require that a) the analyte is determined, that b) independently and by quite a different procedure, creatinine is determined and that c) the results are expressed as CDP/creatinine ratios, unless 24 hour urine collections would be available. It is only the CDP/creatinine ratios, which allow for meaningful comparisons of data from one measurement to the other in and between individuals or patient groups.

The collagen molecules, produced and secreted by the osteoblast are subject to intra- and extra-cellular posttranslational modifications. Especially, the N- and/or C-terminal portions of collagen are subject to extracellular, intra- and intermolecular cross-linking. Tri-valent crosslinks originating from three lysine or hydroxylysine residues are most prominent. Relevant for the embodiments according to this invention is the fact that as a result of bone resorption small collagen peptide residues from the N-or C-terminal portions of the collagen molecule - still containing a cross-linking structure - are found. In the case of the C-terminal telopeptides most of the fragments contain twice the so-called 8-AA sequence Glu-Lys*-Ala-His-Asp**-Gly-Gly-Arg (Lys* may be part of a cross-linking structure; Asp** may be linked to Gly by regular peptide linkage (=α-8AA) or by an iso-peptide bond (=β-8AA). Most CTX fragments therefore are either composed of twice α-8AA, twice β-8AA or, one each, α-8AA and β-8AA.

Specific detection e.g. of CTX containing twice β-8AA is possible and has been described in PCTIWO 98/26286. All the essential features of the sandwich immunoassay to detect CTX based on two monoclonal antibodies are described in WO 98/26286 and the skilled artisan will find all essential technical advice there for carrying on such or similar assays. The assay disclosed works in urine as well as in serum samples. When urine is used, correction for variance in urine concentration has to be made by calculating the CrossLaps™/creatinine ratio. Since neither of the two monoclonal antibodies 1103 or F 12 described and used there, appears to be able to bind twice to the same β-8AA/β-8AA CTX molecule. Either one is appropriate as masbp or as detection reagent or vice versa in the methods and devices according to the present invention.

As described in PCT WO 96/12193 the ratios of so-called α-8AA C-terminal CDPs (α-CTX) to the so-called β-8AA C-terminal CDPs (β-CTX) appear to represent a significant improvement for the assessment of bone turnover based on measurement of CDPs.

However, both α-CTX as well as β-CTX have to be measured independently. Since both can be measured from the same sample, creatinine correction is not a must. However, again and still, at least two independent measurements are required, both measurements being subject to variations during measurement and subject to assay variations from lot-to-lot of the at least two products used.

Somewhat in analogy to PCT WO 96/04554 (the calcium + creatinine test strip discussed above) a device for the simultaneous measurement of both, the concentration of Osteomark™ as well as the concentration of creatinine has quite recently been developed. J.Blatt et al., describe such a device in Clinical Chemistry 44;9,p2051+2052, 1998. This article is entitled "A Miniaturized, Self-contained, Single-use, Disposable Assay Device for the Quantitative Determination of the Bone Resorption Marker NTX in Urine". This (single-use!) handhold device also contains all the equipment necessary to measure both the signals developed by the NTX-specific reaction as well as by the creatinine determination, to calculate the ratio of NTX/creatinine and to display the rationed result.

The method according to the present invention does largely improve on any diagnosis of urinary analytes by directly correcting for sample dilution in one and the same measurement. Independent measurement of creatinine or another reference molecule is no longer required.

The inventive method for the assessment of analyte to reference molecule-ratios (according to key principle I is characterised in that while a sample passes through a device (a) a modified analyte-specific binding partner, which is bound to the reference molecule or a mimetic thereof (I) is released by the reference molecules in the sample and (II) a fraction of masbp forms analyte/masbp-complexes with analyte molecules present in the sample, (b) analyte not bound to masbp in (a (II)) is removed, (c) the complexes formed between analyte and masbp are measured, and, (d) the values measured in (c) are correlated to the values of a calibration curve for analyte to reference molecule ratios. Such calibration or standard curve preferably is independently developed, e.g., when manufacturing and standardising the inventive device, and is used to assess the analyte to reference molecule ratio of the sample.

In one preferred embodiment the urinary analyte will be any form of CDP , preferably NTX or CTX and the result will be reported using creatinine as a reference molecule.

In another preferred embodiment the urinary analytes will be drugs and drug-derivatives.

Using the method of the invention not qualitative (as with test strips of the state of the art) for individual analytes but rather (at least semi-) quantitative data for analyte to reference molecule ratios will be obtained.

The following examples will describe some features of the present invention. The skilled artisan will appreciate that there exist many alternative routes to make use of the basic principles as disclosed in the present invention, as shown in the Figures as and detailed in the Examples.

### Examples

### Example 1: Determination of estrone-3-glucuronide (E3G) to testosterone-17-glucuronide (T17G) ratio

### a) Preparation of monoclonal antibodies to E3G and to T17G

Monoclonal antibodies are prepared and isolated according to the procedures described in WO 95/13543. For both steroids, essentially the same procedures are used. In short: The steroid (40mg) in 1 ml dimethyl formamide is added to two molar equivalents of tri-N-butylamine. After 5 min at room temperature one molar equivalent of isobutyl chloroformate is added. The solution is stirred for 60 min at 4°C, to yield "activated steroid". Bovine serum albumin (BSA) is used as a carrier and coupling of the steroid to BSA is performed at pH 8.0 at 4°C for about 4 hours. Unreacted steroid is removed by dialysis. The BSA-steroid-conjugate is used to repeatedly immunise mice and monoclonal antibodies are generated and selected according to standard procedures.

### b) Coupling of T17G to the anti-E3G monoclonal antibody

T17G is activated as described above and coupled to the purified anti-E3G antibody following the procedure described above for conjugation to BSA. The conjugate, which represents the modified analyte-specific binding partner is purified by dialysis.

### c) Labelling of masbp with colloidal gold particles

Gold sol, was prepared by the hydroxylamine mediated reduction of tetrachloroauric acid in water onto seed gold particles. This procedure is described in the literature: Turkevich, J. et al., Discussions of the Faraday Society, No. 11, p 55-74. Gold sol is coated with aminodextran of 40 000 D and masbp is coupled to the dextran-coated gold particles with (1-ethyl-3(3-dimethylaminopropyl)-carbodiimid (EDAC).

### d) Test strip device

Nitrocellulose is used as material to manufacture compartment I, compartment II, the detection zone and, if required, other areas requiring strong binding of molecules.
For production of Compartment I-material nitrocellulose is coated with antibodies to T17G. Binding sites not occupied by anti- T17G antibody are blocked by addition of inert proteins, e.g. BSA or casein. Material of compartment I is the used to immunologically bind the gold-labelled masbp of example 1 c). The test strip material of compartment I is incubated with a n access of gold-labelled masbp. Unbound masbp is removed and the material of compartment I manufactured into the device to be in direct contact with compartment II and allowing for good liquid flow.
Compartment II is densely coated with antibody to E3G. This antibody does not significantly bind to E3G which already is bound to masbp. The detection zone is manufactured to contain E3G. Two designs have been used (a) a streptavidin strip, coated with biotinylated E3G and (b) direct coating with a BSA-E3G-conjugate as prepared in 1a).
A safety zone or strip manufactured to contain colloidal gold-particles conjugated with T17G and bound via the anti-T17G antibody already used in compartment I may be optionally included into the device.

### Example 2: Determination of the ratio of collagen degradation product(s) to creatinine

### a) Production of anti-creatinine antibodies

Specific antibodies to creatinine are produced as described in EP 864863. In brief:
In order to couple creatinine to other molecules, like carrier proteins or antibodies, it has to be activated. Creatinine is first reacted with 4-bromo-butyric acid-ethylester to yield 4-(2-imino-3-methyl-5-oxo-imidazolidin-1-yl)-butyric acid ethyl ester-hydrobromide. This compound is neutralised by potassium hydroxide (KOH) is then used to generate 4-(2-imino-3-methyl-5-oxo-imidazolidin-1-yl)-butyric acid amide. The activated 4-(2-imino-3-methyl-5-oxo-imidazolidin-1-yl)-butyric acid amide is coupled to keyhole limpet hemocyanine using (1-ethyl-3(3-dimethylaminopropyl)-carbodiimid (EDAC). This conjugate is used to immunise mice. Monoclonal antibodies are generated and selected according to standard procedures. Especially following procedures given in EP 864863.

### b) Production of antibodies with specificity for collagen degradation products

Antibodies to NTX are obtained according to procedures described in the patent literature which has been discussed in the "detailed description" section. To carry out the present invention clone 1H11, as deposited under accession number HB 10 611 with the American Type Culture Collection is used. Commercial use is only possible with permission or license from Ostex International Inc., Seattle.
Antibodies to C-terminal telo-peptides are produced using synthetic peptide antigens. Synthesis of the sequence Glu-Lys-Ala-His-Asp-Gly-Gly-Arg (called 8AA; usually two of these peptide chains are regularly found in CTX) is performed according standard solid phase synthesis procedures (e.g. the "Merrifield procedure"). Either α-8AA or f3-8AA can be synthesised. Methods to conjugate 8AA to a carrier molecule, to immunise laboratory animals, to screen for appropriate antibodies and to select the required binding pairs are given in the relevant patent literature.

In order to perform the present invention, e.g. to determine α-CTX- or the β-CTX to creatinine ratio several approaches are possible. Most preferred, however, is the use of monoclonal antibodies specific either for α- or for β-8AA iso-form of this peptide. It has been shown that the monoclonal antibody A7 is specific for α-BAA, that monoclonal antibody 6E reacts with both form of 8AA, whereas monoclonal antibodies 1103 and F12 are specific for β-8AA and even are capable of forming a sandwich in assays for β-8AA/P-8AA C-telopeptides (WO/98/26286). All these antibodies may be used in the a test device set up according to the competitive immunoassay principle as described below. Antibodies 1103 and F12 are also used in a sandwich immunoassay procedure which is described in Example 3

### c) Production of the creatinine modified analyte-specific binding partner

Creatinine is activated as described in example 2a and the activated 4-(2-imino-3-methyl-5-oxo-imidazolidin-1-yl)-butyric acid amide is coupled to purified immunoglobulin of monoclonal antibody 1H11. Creatinine not conjugated to the monoclonal antibody is removed by dialysis.

### d) Gold-labelling of the modified analyte-specific binding partner

Dextran-coated colloidal gold particles are prepared as described under example 1 c). Coupling of masbp (from 2c) to the dextran-coated colloidal gold particles is performed using 1-ethyl-3(3-dimethylaminopropyl)-carbodiimid (EDAC).

### e) Test strip device for determination of CDP to creatinine ratio (competitive)

The test strip material of compartment I is coated with antibodies to creatinine. Coating is performed by direct absorption on nitrocellulose strips. In case less sticky materials are used coating is either performed by use of 1-ethyl-3(3-dimethylaminopropyl)-carbodiimid (EDAC) or by indirect attachment of biotinylated anti-creatinine antibody to test strip material coated with streptavidin. Binding sites not occupied by anti-creatinine antibody are blocked by addition of inert proteins, e.g. BSA or casein. Anti-creatinine-coated material is then used to immunologically bind the gold-labelled masbp of example 2d). It is incubated with an access of gold-labelled masbp. Unbound masbp is removed and the material is used as compartment I of a test strip device.

Compartment II is densely coated with antibody 1 H11. This antibody does not significantly bind to NTX which already is bound to the masbp derived from the same antibody.
The detection zone is manufactured to contain the NTX under investigation. Coating of NTX is either performed via a streptavidin strip, using biotinylated CDP-peptide or with a BSA-CDP-peptide-conjugate, prepared by coupling with (1-ethyl-3(3-dimethylaminopropyl)-carbodiimid (EDAC) or by use of a heterobifunctional reagent e.g. according to the MH/SH-chemistry principle.
Only those (labelled) masbp-molecules will bind to the detection zone and can be detected, which are not in form of an analyte to masbp complex. The signal generated at the detection zone is indirectly correlated to the CDP-creatinine ratio.

In pilot experiments material of compartment I (5 mm x 1 cm), material of compartment II (5 mm x 1 cm) and NTX-detection strip (5 mm x 2 mm) are placed in close contact to allow for lateral flow of liquid onto a plastic support. Whatman 3M® filter paper is used to facilitate liquid flow though the test strip set-up by bringing the detection strip in contact with this material.
First morning void urine samples from a healthy man, a patient with active Paget's disease, and an osteoporotic patient under therapy with Alendronate® (10 µl each) are applied to most distant edge (opposite to the filter paper) of such prototype devices. Liquid flow is followed by eye. Once the sample has been taken up by the device 20 µl of PBS/Teen-buffer (PBS/Tween = phosphate buffered saline pH 7.5 containing 0.05% Tween® 20) is added to the distant edge and reaction stopped once the buffer has been taken up by the device.

The read-out for the healthy man is set to 100%. The signal obtained with the urine sample from the pagetic patient was 39% of the normal signal whereas, the urine sample of bisphosphonate treated patient was 180% of the healthy control.

Since signal height is indirectly correlated to the NTX to creatinine ratio the former result is indicative for high bone turn-over, whereas the latter is indicative for low bone turn-over and in this special case for successful anti-resorptive treatment.

### Example 3: Determination of the of β-8AA/β-8AA-C-terminal CDP (= β-8AA x β-8AA-CTX ) to creatinine ratio in a sandwich-type assay format

### a) Reagents known from example 2

Synthetic peptides, anti-creatinine antibodies, monoclonal antibodies from clones 1103 and F12 both specific for β-8AA, and procedures for activation or conjugation of creatinine are known from example 2.

### b) Clone 1103 as modified analyte-specific binding partner

Immunoglobulin of monoclonal antibody clone 1103 is produced and purified according to standard procedures. Purified IgG is coupled to creatinine as described in example 2. Various stoichiometries between immunoglobulin and creatinine are tested and the optimal conjugate is selected for production of compartment I.

### c) Monoclonal F 12 as detection reagent

Monoclonal antibody F 12 is cleaved to F (ab) or F (ab)₂-fragments and labelled with colloidal gold according to well-established standard procedures, e.g. as described in Tijssen, 1995, or J.Beesley, "Colloidal gold: A New Perspective for Cytochemical marking", Microscopy handbooks 17, Oxford University Press, 1989.

### d) Test strip device for determination of the of β-8AA/β-8AA-C-terminal CDP to creatinine ratio in a sandwich-type assay format

Compartment I is manufactured to contain creatinine-labelled 1103-antibody from example 3b). This antibody has the special property that it is not capable of forming a sandwich containing twice F12 and the analyte, rather it binds to only one of the two β-8AA-sequences of the analyte which is the twice β-8AA-CTX.
Detection of masbp-analyte-detection-reagent complexes in the detection zone is performed by using a detection zone coated with anti-mouse-IgG(Fc-region)-antibody. This way only 1103 is bound and the signal generated due to presence of labelled F12 in a sandwich is directly correlated to the β-8AA/β-8AA-CTX to creatinine ratio in the sample analysed.

### e) Conduct of pilot assays

Material of compartment I (5 mm x 1 cm), material of compartment II (5 mm x 1 cm), antimouse-lgG(Fc-region)-detection strip (5 mm x 2 mm) are placed in close contact to allow for lateral flow of liquid onto a plastic support. Whatman 3M® filter paper is used to facilitate liquid flow though the test strip set-up by bringing the detection strip in contact with this material.
First morning void urine samples from a healthy man, a patient with active primary hyperparathyroidism, and an osteoporotic patient under therapy with Alendronate® (10µl each) are applied to most distant edge of such prototype devices. Once the sample has been taken up by the device 20 µl of PBS/Teen-buffer is added and reaction stopped once this has been taken up by the device. The detection zone is incubated with the labelled detection reagent, washed and the label bound is measured.

The read-out for the healthy man is set to 100%. The signal obtained with the urine sample from the patient with primary hyperparathyroidism was 231 % of the normal signal whereas, the urine sample of bisphosphonate treated patient was 43% of the healthy control. Since signal height is directly correlated to the β-8AA/β-8AA-CTX to creatinine ratio the former result is indicative for high bone resorption, whereas the latter is indicative for low bone resorption and in this special case for successful anti-resorptive treatment.

With the methods and examples given at hand the skilled artisan will be able to vary the concepts according to his special requirements. Antibodies, from different species, special IgG-subclasses, special antibodies fragments, antibody derivatives, e.g. bearing biotin or digoxigenin, non-antibody binding partners, like lectins, receptors, enzymes and so one may be used to perform the disclosed procedure for determining concentrations of any analyte in relation to an appropriate reference molecule, wherein the appropriate reference molecule will be selected to best match the source of samples used.

## Claims

1. Device for measurement of analyte to reference molecule ratio(s), comprising at least
(a) a first compartment I comprising
(I) a solid support coated with a reference molecule or mimetic thereof
(II) a modified analyte-specltic binding partner (masbp) capable of specifically binding to the analyte and being modified that it binds to said reference molecule or said mimetic thereof said masbp being bound via said reference molecule or mimetic thereof to compartment I in a way that it can be released by reference molecules in the sample, and,
(b) a second compartment **II,** comprising means of removing any analyte not bound to said masbp.

2. Device for measurement of analyte to reference molecule ratio(s), comprising at least
(a) a first compartment I comprising
(I) a solid support coated with a binding partner for the reference molecule,
(II) a modified analyte-specific binding partner capable of binding to the analyte and being modified to carry the reference molecule or mimetics thereof, this modified analyte-specific binding partner being bound to the reference molecule partner of (I) and capable of being released by the reference molecules of the sample, and
(b) a second compartment 11, comprising means of removing any analyte not bound to said modified analyte-specific binding partner.

3. Device according to claims 1 or 2 additionally comprising means for detection of complexes between analyte and modified analyte-specific binding partner.

4. Device according to any of claims 1 to 3 additionally containing a control zone, indicating that sufficient sample to release all masbp has passed through the device.

5. Device according to any of claims 1 to 4 **characterised in that** the above compartments are part(s) of a test strip.

6. A method for the assessment of analyte to reference molecule-ratios **characterised in that** while the sample passes through a device according to claim 1
(a) (I) the modified analyte-specific binding partner is released by the reference molecules in the sample from said first compartment and (II) a fraction of masbp forms analyte/masbp-complexes with analyte molecules present in the sample,
(b) analyte not bound to masbp in (a (II)) is removed in said second compartment.
(c) the complexes formed between analyte and masbp are measured, and,
(d) the values measured in (c) are correlated to the values of a calibration curve for analyte to reference molecule ratios.

7. A method for the assessment of analyte to reference molecule-ratios **characterised in that** while the sample passes through a device according to claim 2
(a) (I) the modified analyte-specific binding partner is released by the reference molecules in the sample from said first compartment and (II) a fraction of masbp forms analyte/masbp-complexes with analyte molecules present in the sample,
(b) analyte not bound to masbp in (a (II)) is removed in said second compartment,
(c) the complexes formed between analyte and masbp are measured, and,
(d) the values measured in (c) are correlated to the values of a calibration curve for analyte to reference molecule ratios.

8. The method according to claim 6 or 7 further **characterised in that** the modified analyte specific binding partner comprises a modified antibody.

9. The method according to any of claims 6 to 8 further **characterised in that** the binding partner of used to remove any analyte not bound to said masbp has essentially the same specificity as the one used as the masbp.

10. The method according to any of claims 6 to 9 further **characterised in that** the analyte/mosbp-complex is detected by use of a second analyte-specific binding partner.

11. The method according to any of claims 6 to 10 further **characterised in that** the second analyte specific binding partner is detectably labelled.

12. The method according to any of claims 8 to 9 further **characterised in that** the modified analyte-specific binding partner is labelled.

## Patentansprüche

1. Vorrichtung zur Messung der Verhältnisse von Analytmolekülen zu Referenzmolekülen, mindestens enthaltend
(a) ein erstes Kompartiment I enthaltend
(I) einen festen Träger, der mit einem Referenzmolekül oder einem Mimetikum desselben beschichtet ist,
(II) einen modifizierten analytspezifischen Bindepartner (masbp), der in der Lage ist, den Analyten spezifisch zu binden und derart modifiziert ist, dass er an besagtes Referenzmolekül oder dessen besagtes Mimetikum bindet, wobei besagter masbp über dieses Referenzmolekül oder über dessen Mimetikum an Kompartiment I derart gebunden ist, dass er durch Referenzmoleküle in der Probe freigesetzt werden kann, und,
(b) ein zweites Kompartiment (II), enthaltend Mittel zur Entfernung von Analyt, welcher nicht an masbp gebunden ist.

2. Vorrichtung zur Messung der Verhältnisse von Analytmolekülen zu Referenzmolekülen, mindestens enthaltend
(a) ein erstes Kompartiment I enthaltend
(I) einen festen Träger, der mit einem Bindepartner für das Referenzmolekül beschichtet ist,
(II) einen modifizierten analytspezifischen Bindepartner (masbp), der in der Lage ist, den Analyten spezifisch zu binden und derart modifiziert ist, dass er das Referenzmolekül oder dessen Mimetikum trägt, wobei besagter masbp über den Bindepartner für das Referenzmolekül an Kompartiment I derart gebunden ist, dass er durch Referenzmoleküle in der Probe freigesetzt werden kann, und,
(b)ein zweites Kompartiment (II), enthaltend Mittel zur Entfernung von Analyt, welcher nicht an masbp gebunden ist.

3. Eine Vorrichtung gemäß der Ansprüche 1 oder 2, ferner Mittel zum Nachweis von Komplexen aus Analyt und modifiziertem analytspezifischem Bindepartner enthaltend.

4. Vorrichtung gemäß einem der vorstehenden Ansprüche 1 bis 3 eine Kontrollzone enthaltend, welche anzeigt, dass genügend Probe, um alle masbp freizusetzen, durch die Vorrichtung gewandert ist.

5. Vorrichtung gemäß einem der vorstehenden Ansprüche 1 bis 4 **dadurch gekennzeichnet, dass** die oben angeführten Kompartimente Teile eines Teststreifens sind.

6. Ein Verfahren zur Untersuchung von Verhältnissen von Analytmolekülen zu Referenzmolekülen, **dadurch gekennzeichnet, dass** während eine Probe durch eine Vorrichtung gemäß Anspruch 1 wandert
(a) (I) der modifizierte analytspezifische Bindepartner durch das Referenzmolekül in der Probe vom ersten Kompartiment I freigesetzt wird und (II) ein Anteil der masbp einen Analyt/masbp Komplex mit Analytmolekülen aus der Probe ausbildet,
(b) Analyt, welcher in (a II)) nicht an masbp gebunden ist, in besagtem zweiten Kompartiment entfernt wird,
(c) die Komplexe gebildet aus Analyt und masbp gemessen, und
(d) die in (c) bestimmten Werte zu den Werten einer Eichkurve für die Verhältnisse von Analyt zu Referenzmolekül korreliert werden.

7. Ein Verfahren zur Untersuchung von Verhältnissen von Analytmolekülen zu Referenzmolekülen, **dadurch gekennzeichnet, dass** während eine Probe durch eine Vorrichtung gemäß Anspruch 2 wandert
(a) (I) der modifizierte analytspezifische Bindepartner durch das Referenzmolekül in der Probe vom ersten Kompartiment I freigesetzt wird und (II) ein Anteil der masbp einen Analyt/masbp Komplex mit Analytmolekülen aus der Probe ausbildet,
(b) Analyt, welcher in (a II)) nicht an masbp gebunden ist, in besagtem zweiten Kompartiment entfernt wird,
(c) die Komplexe gebildet aus Analyt und masbp gemessen, und
(d) die in (c) bestimmten Werte zu den Werten einer Eichkurve für die Verhältnisse von Analyt zu Referenzmolekül korreliert werden.

8. Verfahren gemäß Anspruch 6 oder 7 ferner **dadurch** charakterisiert, dass der modifizierte analytspezifische Bindepartner einen Antikörper enthält.

9. Verfahren gemäß einem der Ansprüche 6 bis 8 ferner **dadurch** charakterisiert, dass der Bindepartner, welcher zur Entfernung von nicht an den masbp gebundenem Analyt eingesetzt wird, im wesentlichen die gleiche Spezifität besitzt, wie derjenige aus dem masbp.

10. Verfahren gemäß einem der Ansprüche 6 bis 9 ferner **dadurch gekennzeichnet, dass** der Analyt/masbp-Komplex mit Hilfe eines zweiten analytspezifischen Bindepartners nachgewiesen wird.

11. Verfahren gemäß einem der Ansprüche 6 bis 9 ferner **dadurch gekennzeichnet, dass** der zweite analytspezifische Bindepartner nachweisbar markiert ist.

12. Verfahren gemäß einem der Ansprüche 6 bis 9 ferner **dadurch gekennzeichnet, dass** der modifizierte analytspezifische Bindepartner nachweisbar markiert ist.

## Revendications

1. Dispositif de mesure de(s) rapport(s) analyte sur molécule de référence, comprenant au moins
(a) un premier compartiment I comprenant
(I) un support solide revêtu d'une molécule de référence ou d' un mimétique de celle-ci,
(II) un partenaire de liaison modifié spécifique d'un analyte (plmsa) pouvant se lier de manière spécifique à l'analyte et être modifié de sorte qu'il se lie à ladite molécule de référence ou audit mimétique de celle-ci, ledit plmsa étant lié par l'intermédiaire de ladite molécule de référence ou dudit mimétique de celle-ci au compartiment I de telle manière qu'il peut être libéré par les molécules de référence dans l'échantillon, et,
(b) un deuxième compartiment II, comprenant des moyens d'élimination de tout analyte non lié audit plmsa.

2. Dispositif de mesure de(s) rapport(s) analyte sur molécule de référence, comprenant au moins
(a) un premier compartiment I comprenant
(I) un support solide revêtu d'un partenaire de liaison de la molécule de référence,
(II) un partenaire de liaison modifié spécifique d'un analyte pouvant se lier à l'analyte et être modifié de manière à porter la molécule de référence ou des mimétiques de celle-ci, ce partenaire de liaison modifié spécifique d'un analyte étant lié au partenaire de la molécule de référence de (I) et pouvant être libéré par les molécules de référence de l'échantillon, et,
(b) un deuxième compartiment II, comprenant des moyens d'élimination de tout analyte non lié audit partenaire de liaison modifié spécifique d'un analyte.

3. Dispositif selon les revendications 1 ou 2, comprenant en outre des moyens de détection de complexes entre l'analyte et le partenaire de liaison modifié spécifique d'un analyte.

4. Dispositif selon l'une quelconque des revendications 1 à 3, comprenant en outre une zone de contrôle indiquant que suffisamment d'échantillon est passé dans le dispositif pour libérer tous les plmsa.

5. Dispositif selon l'une quelconque des revendications 1 à 4, **caractérisé en ce que** les compartiments ci-dessus constituent une(des) partie(s) d'une bandelette réactive.

6. Procédé d'évaluation des rapports analyte sur molécule de référence, **caractérisé en ce que**, alors que l'échantillon passe dans un dispositif selon la revendication 1,
(a) (I) le partenaire de liaison modifié spécifique d'un analyte est libéré par les molécules de référence dans l'échantillon dudit premier compartiment et (II) une fraction de plmsa forme des complexes analyte/plmsa avec des molécules d'analytes présentes dans l'échantillon,
(b) un analyte non lié à un plmsa en (a (II)) est éliminé dans ledit deuxième compartiment,
(c) les complexes formés entre un analyte et un plmsa sont mesurés, et,
(d) les valeurs mesurées en (c) sont corrélées avec les valeurs d'une courbe d'étalonnage pour les rapports analyte sur molécule de référence.

7. Procédé d'évaluation des rapports analyte sur molécule de référence, **caractérisé en ce que**, alors que l'échantillon passe dans un dispositif selon la revendication 2,
(a) (I) le partenaire de liaison modifié spécifique d'un analyte est libéré par les molécules de référence dans l'échantillon dudit premier compartiment et (II) une fraction de plmsa forme des complexes analyte/plmsa avec des molécules d'analytes présentes dans l'échantillon,
(b) un analyte non lié à un plmsa en (a (II)) est éliminé dans ledit deuxième compartiment,
(c) les complexes formés entre un analyte et un plmsa sont mesurés, et,
(d) les valeurs mesurées en (c) sont corrélées avec les valeurs d'une courbe d'étalonnage pour les rapports analyte sur molécule de référence.

8. Procédé selon la revendication 6 ou 7, **caractérisé en outre en ce que** le partenaire de liaison modifié spécifique d'un analyte comprend un anticorps modifié.

9. Procédé selon l'une quelconque des revendications 6 à 8, **caractérisé en outre en ce que** le partenaire de liaison utilisé pour éliminer tout analyte non lié audit plmsa possède essentiellement la même spécificité que celui utilisé comme plmsa.

10. Procédé selon l'une quelconque des revendications 6 à 9, **caractérisé en outre en ce que** le complexe analyte/plmsa est détecté grâce à l'utilisation d'un deuxième partenaire de liaison spécifique d'un analyte.

11. Procédé selon l'une quelconque des revendications 6 à 10, **caractérisé en outre en ce que** le deuxième partenaire de liaison spécifique d'un analyte est marqué de manière détectable.

12. Procédé selon l'une quelconque des revendications 8 ou 9, **caractérisé en outre en ce que** le partenaire de liaison modifié spécifique d'un analyte est marqué.
